# EUROPEAN PATENT APPLICATION

(11) **EP 3 919 905 A1**
(43) Date of publication of application: **08.12.2021**
(21) Application number: 20748848.7
(22) Date of filing: 29.01.2020
(51) Int. Cl.: G01N 33/53, G01N 33/543, G01N 33/569

(54) **IMMUNOCHROMATOGRAPHIC TEST KIT FOR EXTRACTING AND MEASURING SUGAR CHAIN ANTIGENS AND CAPABLE OF CONTROLLING ANALYTE DEVELOPMENT**

(30) Priority: 29.01.2019 JP 2019012912
(71) Applicant: Denka Company Limited, Tokyo 103-8338 (JP)
(72) Inventor: KATO Daisuke, Gosen-shi, Niigata 959-1695 (JP); MURAMATSU Shino, Gosen-shi, Niigata 959-1695 (JP)
(74) Representative: Dietz, Christopher Friedrich
(86) International application number: PCT/JP2020/003064
(87) International publication number: WO 2020/158763

(57) **Abstract**

The present invention aims to provide: an immunochromatographic test kit for controlling the development of a specimen on an immunochromatographic test strip and for appropriately controlling a treatment using an acid reagent, a nitrite and a neutralizing reagent; and an immunochromatography method using the kit. The immunochromatographic test kit includes: (i) a nitrite solution or an acid solution; and (ii) an immunochromatographic test strip for extracting and measuring a sugar chain antigen in a specimen, comprising: a sample pad to which a specimen mixed with the nitrite or acid solution is added; a label region comprising a labeled antibody obtained by labeling an antibody against the sugar chain antigen; and a detection region on which an antibody against the sugar chain antigen is immobilized, wherein an antibody-sugar chain antigen-labeled antibody complex is formed in the detection region to measure the sugar chain antigen, and wherein the immunochromatographic test strip has a region impregnated with a neutralizing reagent upstream of the label region, and further has, upstream of the region impregnated with the neutralizing reagent, a region impregnated with a solid acid reagent when the specimen mixed with the nitrite is used, or a region impregnated with the nitrite when the specimen mixed with the acid solution is used, and wherein the nitrite or acid solution comprises one or more selected from the group consisting of polyoxyethylene octyl phenyl ether, a quaternary ammonium compound, phosphoric acid, sodium hydroxide, N-acetyl-L-cysteine, NaCl, PVA, BSA and NaBr.

## Description

### Technical Field

The present invention relates to an immunochromatographic test kit for extracting and measuring a sugar chain antigen, which is capable of extracting a sugar chain antigen with nitrous acid on an immunochromatographic test strip.

### Background Art

A majority of rapid diagnostic agents involving an immunochromatography method as a principle have been broadly used as a means for promptly and simply measuring viral or bacterial infection and determining a treatment plan therefor.

In the case of such rapid diagnostic agents involving common immunochromatography as a principle, a specimen is suspended in a specimen suspension solution, and the suspension is then supplied to an immunochromatographic test strip, so that the measurement can be carried out rapidly and simply.

For detection of microorganisms belonging to genus *Streptococcus,* such as group A β-hemolytic streptococcus and intraoral streptococcus, it is necessary to extract and measure sugar chain antigens.

For example, a method has been reported in which sodium nitrite and a neutralizing reagent are already included in an immunochromatographic test strip, so that a nitrous acid extraction treatment can be carried out on the immunochromatographic test strip simply through the operation of suspending a specimen in an acid solution such as acetic acid and to supply the suspension to the immunochromatographic test strip (Patent Literature 1).

In addition, a method has been reported in which an acid reagent and a neutralizing reagent have been already included in to an immunochromatographic test strip, and a specimen is suspended in a nitrite and supplied to the immunochromatographic test strip.

Further, a method has been reported in which nitric acid extraction treatment of a sugar chain antigen is efficiently carried out on an immunochromatographic test strip (Patent Literatures 2 to 4).

### Citation List

### Patent Literature

Patent Literature 1: International Publication WO2005/121794
Patent Literature 2: JP Patent Publication No. 2018-151329 A
Patent Literature 3: JP Patent Publication No. 2018-151330 A
Patent Literature 4: JP Patent Publication No. 2018-151331 A

### Summary of Invention

### Technical Problem

Most of currently commercially available group A β-hemolytic Streptococcus (Strep A) antigen detecting reagents utilizing an immunochromatography method as a principle employ a technique wherein acidic nitrous acid generated by mixing a nitrite solution (R1) with an acid solution (R2) is neutralized and reacted with a neutralizing reagent (R3) present in a dry state on a immunochromatographic test strip (test strip). However, to make the specimen preparation with a single step, a technique is also known to eliminate fresh preparation of mixing R1 and R2, and an extraction process by applying either R1 or R2 onto a pad. For such a Strep A antigen detecting reagents, which can be operated with a single step, an extraction process is conducted on a test device, so it is essential to ensure that extraction occurs for a predetermined time period after applying a specimen. However, when a specimen with a high viscosity was used, a phenomenon where a time period for the specimen to remain on a test device tended to be too long. This caused problems such as (1) a sample not developing; (2) even though the development occurs, a delay of development reduces the sensitivity at 5 minutes after the start of measurement; (3) even though the development occurs, a non-specific reaction is occured.

It is an object of the present invention to provide an immunochromatographic test kit, which controls the development of a specimen on an immunochromatographic test strip and appropriately controls a treatment with an acid reagent and a nitrite, and a neutralizing reagent; and an immunochromatography method using the kit.

### Solution to Problem

The present inventors have conducted intensive studies regarding a method of controlling the development of a specimen on an immunochromatographic test strip and appropriately control treatments of a specimen with an acid reagent and a nitrite, and a neutralizing reagent.

As a result, the present inventors have found that adding a compound such as a surfactant, salts, a chemical synthetic substance (PVA), a proteinaceous substance (BSA) to a specimen suspension solution in advance shortens a time needed for development initiation leading to earlier determination of the lines also alleviate a non-specific reaction, thereby completing the present invention.

That is, the present invention is as follows.
[1] An immunochromatographic test kit comprising:
   (i) a nitrite solution or an acid solution; and
   (ii) an immunochromatographic test strip for extracting and measuring a sugar chain antigen in a specimen, comprising: a sample pad to which a specimen mixed with the nitrite or acid solution is added; a label region comprising a labeled antibody obtained by labeling an antibody against the sugar chain antigen; and a detection region on which an antibody against the sugar chain antigen is immobilized, wherein an antibody-sugar chain antigen-labeled antibody complex is formed in the detection region to measure the sugar chain antigen, and wherein the immunochromatographic test strip has a region impregnated with a neutralizing reagent upstream of the label region, and further has, upstream of the region impregnated with the neutralizing reagent, a region impregnated with a solid acid reagent in case the specimen mixed with the nitrite is used, or a region impregnated with the nitrite in case the specimen mixed with the acid solution is used,
   wherein the nitrite or acid solution comprises one or more selected from the group consisting of polyoxyethylene octyl phenyl ether, a quaternary ammonium compound, phosphoric acid, sodium hydroxide, N-acetyl-L-cysteine, NaCl, PVA, BSA and NaBr.
[2] The immunochromatographic test kit according to [1], wherein the region impregnated with the solid acid reagent or the nitrite is present on the sample pad.
[3] The immunochromatographic test kit according to [1] or [2], wherein the solid acid reagent is selected from the group consisting of malonic acid, malic acid, maleic acid, citric acid, and tartaric acid.
[4] The immunochromatographic test kit according to any one of [1] to [3], wherein the neutralizing agent is tris(hydroxymethyl)aminomethane or sodium hydroxide.
[5] The immunochromatographic test kit according to any one of [1] to [4], wherein the sugar chain antigen is a sugar chain antigen of protozoa, fungi, bacteria, mycoplasma, rickettsia, chlamydia, or virus.
[6] The immunochromatographic test kit according to any one of [1] to [5], wherein the polyoxyethylene octyl phenyl ether is Triton X, and the quaternary ammonium compound is benzalkonium chloride or benzethonium chloride.
[7] A method for measuring a sugar chain antigen in a specimen by an immunochromatographic method using an immunochromatographic test kit of any one of [1] to [6], comprising:
   mixing the specimen with a nitrous acid solution when the immunochromatographic test strip has a region impregnated with a solid acid reagent, or mixing the specimen with an acid solution when the immunochromatographic test strip has a region impregnated with a nitrite, and adding the mixture to a sample pad of the immunochromatographic test strip,
   wherein, in the immunochromatography method, the sugar chain antigen is extracted from the specimen by the action of nitrous acid generated through a reaction of the nitrite with the solid acid reagent in the region impregnated with the solid acid reagent or the region impregnated with the nitrite, the acid solution containing the sugar chain antigen is neutralized in a region impregnated with a neutralizing reagent, and an antibody-sugar chain antigen-labeled antibody complex is formed in a detection region, and,
   wherein the immunochromatography method controls a speed or a direction of development of the specimen on the immunochromatographic test strip and controls treatments of the acid, the nitrite and the neutralizing reagent to detect sugar chain antigens in specimens.

The present description includes the contents disclosed in Japanese Patent Application No. 2019-012912, which is a priority document of the present application.

### Advantageous Effects of Invention

Addition of any of a surfactant, salts, N-acetyl-L-cysteine, a proteinaceous substance such as BSA, or other chemical synthetic substances to the nitrite solution (R1) or the acid solution can provide a device that prevents a lack of sensitivity caused by a development defect or a development delay, or a non-specific reaction even when a highly viscous pharyngeal swab specimen is used.

### Brief Description of Drawings

[Figure 1] Figure 1 is a schematic figure showing a structure of an immunochromatographic test strip (two-pad test strip) having a solid acid reagent region and a neutralizing reagent region, wherein the solid acid reagent region serves as a sample pad.
[Figure 2] Figure 2 is a schematic figure showing a structure of an immunochromatographic test strip (single-pad test strip) having a solid acid reagent region and a neutralizing reagent region, wherein the solid acid reagent region and the neutralizing reagent region serves as a sample pad.
[Figure 3] Figure 3 is a schematic figure showing a structure of an immunochromatographic test strip (two-pad test strip) having a solid acid reagent region and a neutralizing reagent region, wherein the solid acid reagent region serves as the neutralizing reagent region and a sample pad is separately present.
[Figure 4] Figure 4 is a schematic figure showing a structure of an immunochromatographic test strip having a solid acid reagent region and a neutralizing reagent region, wherein a PET sheet is adhered between the solid acid reagent region and the neutralizing reagent region.
[Figure 5] Figure 5 is a schematic figure showing a structure of an immunochromatographic test strip having a solid acid reagent region and a neutralizing reagent region, wherein a PET sheet is adhered between the solid acid reagent region and the neutralizing reagent region and it also depicts distances between parts.

### Description of Embodiments

Hereafter, the present invention will be described in details.

The present invention relates to an immunochromatographic test kit, which simplifies a nitrous acid extraction treatment of a sugar chain antigen so as to carry out the treatment on an immunochromatographic test strip, and is able to promptly and accurately measure the sugar chain antigen as an analyte to be detected.

The immunochromatographic test kit comprises: an immunochromatographic test strip impregnated with a nitrite or an acid reagent; and a nitrite solution or an acid solution.

An immunochromatographic test strip comprises: a support having a detection region with an immobilized antibody (Antibody 1) that captures an analyte to be detected (antigen, etc.); a label region having a movable labeled antibody (Antibody 2); a sample pad to which a specimen is added; an absorption band that absorbs a developed specimen solution; a backing sheet for adhering these members to one another; and the like.

The immunochromatographic test strip of the present invention may be accommodated in a storing vessel. The storing vessel can prevent the degradation of the test strip, which is caused by, for example, ultraviolet rays or moisture contained in the air. Moreover, in the case of treating a specimen sample having contamination or infectivity, such a storing vessel can prevent a user performing an assay from being contaminated or infected with the specimen or the sample. For instance, a resin-made case having a suitable size may be used as a storing vessel, and the device of the present invention may be accommodated in the case. Alternatively, the surface of a test strip having an antigen or an antibody immobilized thereon may be coated with a resin-made film or the like (PET sheet). There is a case where a storing vessel and a test strip accommodated therein are collectively referred to as an immunochromatographic device.

It is to be noted that the number of detection regions and the type of a labeled antibody contained in the label region are not limited to one, and that, by using antibodies corresponding to a plurality of analyte to be detected, two or more antigens can be measured on a single test strip.

The support is a material having the property of immobilizing an antibody used to capture a substance to be detected (an antigen), and also, it does not prevent the movement of a liquid in the horizontal direction. Preferably, the support is a porous thin film (membrane) having a capillary action, and is a material capable of transporting a liquid and components dispersed therein according to absorption. The material used for the support is not particularly limited, and examples thereof include cellulose, nitrocellulose, cellulose acetate, polyvinylidene difluoride (PVDF), glass fiber, nylon, and polyketone. Among these materials, a thin film or a membrane of nitrocellulose is more preferable. A membrane having an antibody immobilized thereon is referred to as an "antibody-immobilized membrane."

The label region is composed of a porous substrate comprising a labeled antibody, and a commonly used glass fiber, non-woven fabric, and the like can be used herein as a material for the substrate. The substrate is preferably a pad having a thickness of approximately 0.3 mm to 0.6 mm, in order that the substrate is impregnated with a large amount of a labeled antibody. A porous substrate that is impregnated with a labeled antibody and then dried is also referred to as a dry pad.

For the labeling of a labeled antibody, enzymes such as alkaline phosphatase or horse radish peroxidase, metal colloids such as gold colloids, silica particles, cellulose particles, magnetic particles, fluorescent particles, colored polystyrene particles, colored latex particles, etc. are used in many cases. When metal colloidal particles or colored particles such as colored polystyrene particles or colored latex particles are used, color is developed by aggregation of these labeling reagents. So, the thus developed color is measured. Particles having antibodies immobilized thereon are referred to as antibody-immobilized particles.

The detection region indicates a region of the support, on which an antibody used to capture a substance to be detected (an antigen) is immobilized. In the detection region, at least one region, on which an antibody used to capture an antigen is immobilized, is provided. The detection region may be comprised in the support, and an antibody may be immobilized on the support.

The sample pad is a site to which a specimen is added, and is a porous material. The sample pad is a site located most upstream of the immunochromatographic test strip. As a material for the sample pad, a commonly used filter paper, glass fiber, non-woven fabric, etc. can be used. In order to use a large amount of specimen in immunoassay, the sample pad is preferably a pad having a thickness of approximately 0.3 mm to 1 mm. The specimen also includes a sample prepared using the specimen, such as a sample obtained by suspending the specimen in another solution.

The absorbent pad is a component for absorbing leftover specimens, which are supplied to the support but are not associated with the reaction in the detection region. As a material for the absorbent pad, a highly water-retainable filter paper, sponge or the like, composed of a common natural polymer, a synthetic polymer or the like can be used. In order to promote the development of a specimen, a highly water-absorbable material is preferably used.

The backing sheet is a component used for adhesion and/or fixation of all of the aforementioned materials, namely, the support, the sample pad, the label region, the absorbent pad and the like, in which these materials are partially overlapped with one another. The backing sheet is not always needed, as long as these materials are arranged and fixed with optimal intervals. However, it is generally preferred to use a backing sheet for convenience of production or use.

In the immunochromatographic test strip of the present invention, a control display region (a member) may be further present. The control display region is a site showing that a test is accurately carried out. For example, the control display region is located downstream of the detection region, and emits signals such as coloration, when a specimen sample passes through the detection region and reaches the control display region. In the control display region, a substance capable of binding to a labeled carrier-bound antibody may be immobilized, or a reagent such as a pH indicator, which changes its color upon arrival of a specimen, may be immobilized. When such a labeled carrier-bound antibody is a mouse monoclonal antibody, an anti-mouse IgG antibody may be used.

The size of an immunochromatographic test strip is not limited. For example, the longitudinal length thereof is from several to more than 10 centimeters and the lateral length thereof is from about several millimeters to several centimeters.

In the test strip of the above-described embodiment, the specimen passes through porous flow channels formed by connecting a series of members, such as the sample pad, the label region, the support, the detection region and the absorption band, with one another. Thus, in this embodiment, all of these components serve as a specimen moving region. There may be an embodiment wherein a specimen moves on an interface without penetrating an interior of a material depending on the quality or form of each component material. However, since the specimen moving region defined in the present description can be an interior or an interface, a test strip of such an embodiment is also included in the scope of the present description.

In the case of measuring a sugar chain antigen in a specimen using the immunochromatographic test strip of the present invention, it is necessary to first extract the sugar chain antigen in the specimen. The extraction of the sugar chain antigen is performed by treating the specimen containing the sugar chain antigen with nitrous acid. The nitrous acid can be generated by mixing a nitrite such as sodium nitrite with an acid, and the specimen containing the sugar chain antigen may be treated with the nitrous acid thus generated. The extracted antigen binds through an antigen-antibody reaction to the antibody immobilized on the immunochromatographic test strip. On this occasion, if an acid remains in the reaction system, the reaction system becomes acidic and the antigen-antibody reaction is inhibited. Thus, it is necessary to neutralize the acid in the reaction system.

In the method of measuring a sugar chain antigen by mixing a nitrite with an acid to generate nitrous acid, extracting the sugar chain antigen from a specimen with the nitrous acid, neutralizing the acid, and then allowing the sugar chain antigen to bind to the antibody immobilized on the immunochromatographic test strip, examples of the method of extracting and measuring a sugar chain antigen include the following methods. In any of the methods, the extraction of the sugar chain antigen with nitrous acid, and the neutralization are performed on the immunochromatographic test strip. In order to perform the extraction of the sugar chain antigen with nitrous acid on the immunochromatographic test strip, the immunochromatographic test strip may be impregnated with an acid reagent or a nitrite. In order to perform the neutralization on the immunochromatographic test strip, the immunochromatographic test strip may be impregnated with a neutralizing reagent.
(A) A specimen is mixed with an acid solution prior to addition of the mixture to a sample pad of the immunochromatographic test strip impregnated with a nitrite and a neutralizing reagent. When the mixture arrives at the region impregnated with the nitrite, the nitrite reacts with the acid to generate nitrous acid, so that a sugar chain antigen in the specimen is extracted. The extract of the sugar chain antigen is neutralized in the region impregnated with the neutralizing reagent on the immunochromatographic test strip, and the sugar chain antigen binds to the antibody immobilized on the immunochromatographic test strip and can thus be detected. In this method, examples of the acid solution to be used include acetic acid, hydrochloric acid, malonic acid, malic acid, maleic acid, citric acid, and tartaric acid.
(B) A specimen is mixed with a nitrite solution prior to addition of the mixture to a sample pad of the immunochromatographic test strip impregnated with an acid reagent and a neutralizing reagent. When the mixture arrives at the region impregnated with the acid reagent, the nitrite reacts with the acid to generate nitrous acid, so that a sugar chain antigen in the specimen is extracted. The extract of the sugar chain antigen is neutralized in the region impregnated with the neutralizing reagent on the neutralizing immunochromatographic test strip, and the sugar chain antigen binds to the antibody immobilized on the immunochromatographic test strip and can thus be detected.

In the immunochromatographic test strip of the present invention for performing the above-described method (A) or (B), an immunochromatographic test strip impregnated with the nitrite upstream from the label region (on the upstream side along the development of the specimen, where the sample pad is present), namely, within the sample pad or between the sample pad and the label region can be used for the above-described method (A). In addition, an immunochromatographic test strip impregnated with the acid reagent within the sample pad or between the sample pad and the label region can be used for the above-described method (B). Note that a solid acid reagent is used as the acid reagent. According to these methods, a substance to be detected in a test sample can be accurately and specifically measured, regardless of the amount of the test sample to be tested.

The region impregnated with a solid acid reagent is referred to as a solid acid reagent region; the region impregnated with an acid reagent is referred to as an acid reagent region; and the region impregnated with a neutralizing reagent is referred to as a neutralizing reagent region.

The solid acid reagent or the nitrite may be impregnated into the sample pad, or may be impregnated into a pad made of a porous material such as a non-woven fabric, which is different from the sample pad, and the obtained solid acid reagent-impregnated porous material or nitrite-impregnated porous material may be disposed between the sample pad and the label region, namely, on the side upstream of the label region. In the above, the region impregnated with the solid acid reagent or the nitrite may or may not make contact with the sample pad or the label region. In the present invention, the region impregnated with a reagent is also referred to as a pad impregnated with a reagent.

The neutralizing reagent is disposed downstream of the region impregnated with the solid acid reagent or the nitrite. The neutralizing reagent may be impregnated into the support; or it may be impregnated into a pad made of a porous material such as a non-woven fabric, which is different from the support, and the obtained neutralizing reagent-impregnated porous material may be disposed between the region impregnated with the solid acid reagent or the nitrite and the label region. That is, a region impregnated with a neutralizing reagent is provided upstream of the label region, and further a region impregnated with a solid acid reagent or a nitrite is provided upstream of the region impregnated with the neutralizing reagent.

In the above, the region impregnated with the neutralizing reagent may or may not make contact with the region impregnated with the solid acid reagent or the nitrite, or the label region.

Examples of the porous material to be used for a region impregnated with a nitrite reagent, an acid reagent or a neutralizing reagent specifically include a filter paper made of cellulose cotton fiber and a glass filter paper made of glass fiber.

The immunochromatographic test strip having a solid acid reagent region or a nitrite region and a neutralizing reagent region has, on the support, a sample pad, a solid acid reagent region or a nitrite region, a neutralizing reagent region, a label region, a detection region, and an absorption band, from the upstream thereof, and the solid acid reagent region or the nitrite region may be located on the sample pad. Moreover, the sample pad, the solid acid reagent region or the nitrite region, the neutralizing reagent region, the label region, the detection region, and the absorbent pad may or may not make contact with a region adjacent thereto.

The solid acid reagent to be used in the present invention is in a solid state at normal temperature and does not volatilize at a high temperature.

Examples of the solid acid reagent that is preferably used in the present invention may include malonic acid, malic acid, maleic acid, citric acid, and tartaric acid.

If an acid with a higher valence, for example, citric acid, is used as a preferred solid acid reagent to be used in the present invention, extraction can be performed with a smaller amount of acid. On the other hand, if acids have the same valence, an acid having a smaller acid dissociation constant, such as maleic acid or tartaric acid, is more efficient.

Also, as a preferred solid acid reagent to be used in the present invention, a reagent that is not colored on the immunochromatographic test strip, and more specifically, preferred is a reagent, which has white color in a dry state or is hardly colored by dry heat or oxidation.

Examples of the nitrite to be used in the present invention include sodium nitrite and potassium nitrite.

The amount of the solid acid reagent or the nitrite to be used in the present invention, namely, the amount of the solid acid reagent or the nitrite impregnated into the immunochromatographic test strip is not particularly limited. In general, the amount thereof is approximately 0.01 µg to 1 mg, and is preferably approximately 0.1 µg to 0.1 mg, with respect to a single immunochromatographic test strip. However, it is preferred to determine such an optimal amount that provides an effect depending on the type of the solid acid reagent or the nitrite to be used, the composition of a specimen suspension, the amount thereof to be added, etc.

In order to impregnate a sample pad or a porous material with the solid acid reagent or the nitrite, the solid acid reagent or the nitrite is once dissolved, and applied and dried. For example, 0.1 to several molars of solid acid reagent or nitrite may be applied and dried.

The neutralizing reagent used in the present invention is in a solid state at normal temperature and does not volatilize at a high temperature. The neutralizing reagent is also referred to as a basic reagent.

Examples of preferred neutralizing reagents to be used in the present invention include Tris base (tris(hydroxymethyl)aminomethane), sodium hydroxide, dipotassium hydrogen phosphate, trisodium citrate, and Good's buffers having a buffering ability in the alkaline range.

The amount of the neutralizing reagent to be used in the present invention, namely, the amount thereof impregnated into the immunochromatographic test strip is not particularly limited. In general, the amount thereof is approximately 0.01 µg to 1 mg, and preferably approximately 0.1 µg to 0.1 mg, with respect to a single immunochromatographic test strip. However, it is preferred to determine and select an optimal amount that is the most effective. Considerations should be given types of the neutralizing reagent used, the composition of specimen suspension solution and the amount thereof to be added, etc.

In order to impregnate a porous material with the neutralizing reagent, the neutralizing reagent may be once dissolved, and the solution may be applied to the porous material and then dried. For example, 0.1 to several molars, preferably 0.5 to several molars of neutralizing reagent may be applied and dried.

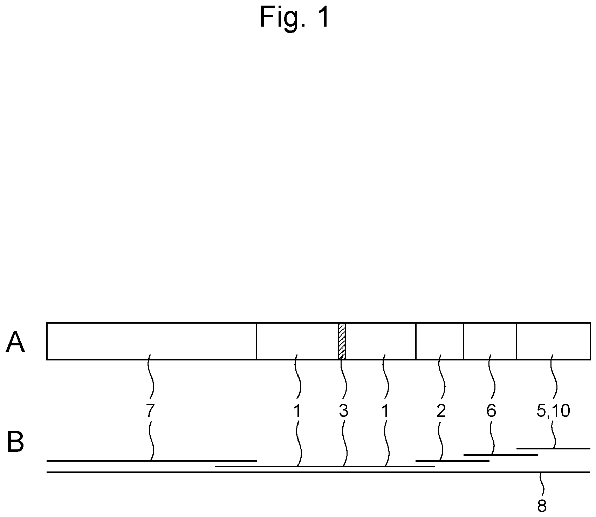

Figures 1 to 3 each show one preferred embodiment of a typical immunochromatographic test strip. The immunochromatographic test strip shown in Figure 1 is an immunochromatographic test strip impregnated with a solid acid reagent and a neutralizing reagent. However, a nitrite may be impregnated instead of the solid acid reagent; and in this case, the resultant is an immunochromatographic test strip impregnated with a nitrite and a neutralizing reagent. That is, in the following explanation on the test strip, a nitrite reagent region may be provided instead of a solid acid reagent region. Those skilled in the art can appropriately design and produce an immunochromatographic test strip impregnated with a nitrite and a neutralizing reagent. It should be noted that the immunochromatographic test strip is not limited to those shown in Figures 1 to 3. In Figure 1, reference numeral 1 denotes a support, reference numeral 2 denotes a label region, reference numeral 3 denotes a detection region, reference numeral 7 denotes an absorbent pad and reference numeral 8 denotes a backing sheet.

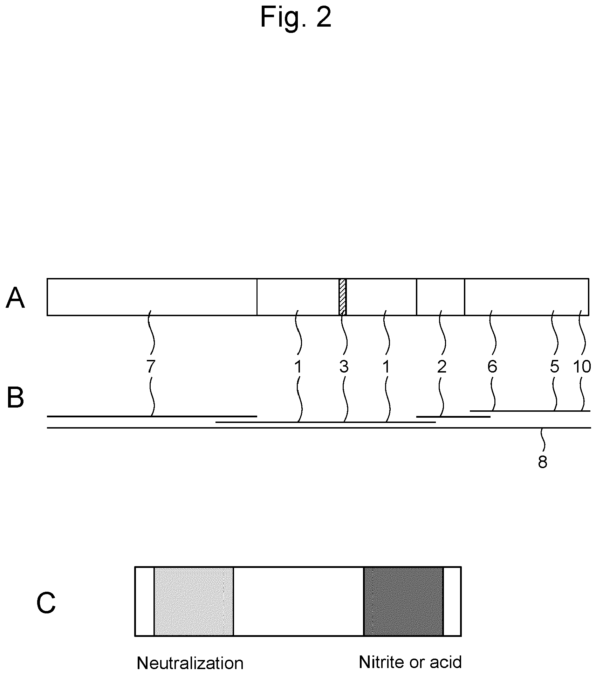

Figures 1 and 2 each show a test strip wherein a solid acid reagent region 5 also serves as a sample pad 10. Figure 1A is a top view and Figure 1B is a cross-sectional view. In the example shown in Figure 1, the solid acid reagent region 5 and/or the neutralizing reagent region 6 is present upstream of the label region 2, and these are overlapped with each other, so that flow channels of continuous lateral flow is formed. In the test strip shown in Figure 1, the solid acid reagent region 5 also serves as a sample pad 10. That is, the solid acid reagent region 5 is present on the sample pad 10. This test strip has the solid acid reagent region 5 and the neutralizing reagent region 6 provided on two separate porous materials (pads), and thus, it is also referred to as a two-pad test strip. A sample pad may further be present upstream of the solid acid reagent region. The solid acid reagent region and the sample pad may be present separately.

In the test strip of Figure 2, a sample pad 10 also serves as a solid acid reagent region 5 and a neutralizing reagent region 6, and the solid acid reagent region 5 and the neutralizing reagent region 6 are present on the sample pad 10. Figure 2A is a top view, Figure 2B is a cross-sectional view, and Figure 2C shows a positional relationship between the neutralizing reagent region 6, and a solid acid reagent region 5 or a nitrite reagent region. This test strip has the solid acid reagent region 5 and the neutralizing reagent region 6 provided on one porous material (pad), so it is referred to as a single-pad test strip.

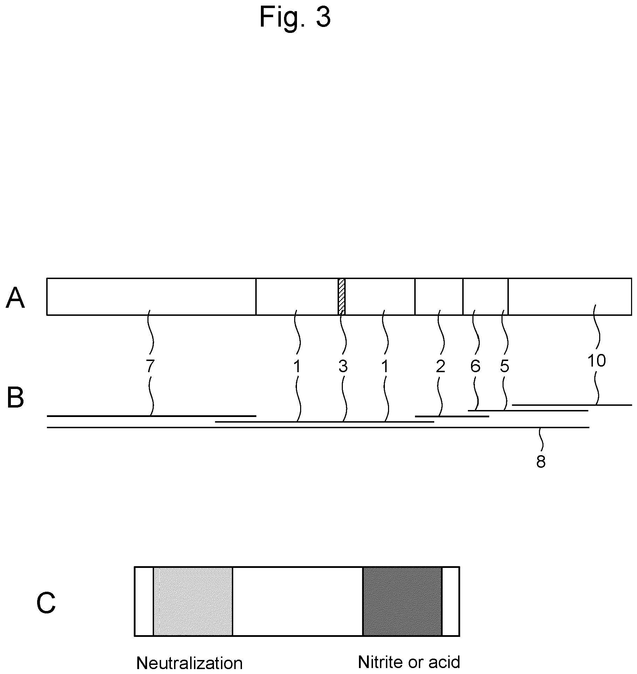

In a test strip of Figure 3, a sample pad 10 is present separately from a solid acid reagent region 5 and a neutralizing reagent region 6; and the solid acid reagent region 5 serves also as the neutralizing reagent region 6 and the sample pad is separately present. The sample pad 10 is impregnated with no reagent and it functions only as a sample pad. Figure 3A is a top view, Figure 3B is a cross-sectional view, and Figure 3C shows a positional relationship between the neutralizing reagent region 6, and the solid acid reagent region 5 or a nitrite reagent region. The test strip of Figure 3 has: a porous material (pad) serving as both a solid acid reagent region and a neutralizing reagent region; and a sample pad, which are provided as two separate porous materials (pads), and therefore it can be referred to as a two-pad test strip. Though not illustrated, three members of the sample pad 10, the solid acid reagent region 5 and the neutralizing reagent region 6 may be present separately.

Measurement is started by bringing a specimen or a sample prepared by use of the specimen into contact with a nitrite solution by mixing to suspend the specimen in the nitrite solution; and adding to the sample 10, the sample pad 10 also serving as the solid acid reagent region 5, or the sample pad 10 serving as both the neutralizing reagent region 6 and the solid acid reagent region 5, where 5 to 100 µL of the specimen and 0.01 to 2 mL of 0.1 to 8 molars of nitrite are mixed with each other, 5 to 200 µL of the mixture may be applied to the sample pad 10, the sample pad 10 serving as the solid acid reagent region 5, or the sample pad 10 serving as both the neutralizing reagent region 6 and the solid acid reagent region 5. Examples of the nitrite include sodium nitrite and potassium nitrite.

A specimen containing a sugar chain antigen as a substance to be detected is supplied to the sample pad 10, the sample pad 10 serving as the solid acid reagent region 5, or the sample pad 10 serving as both the neutralizing reagent region 6 and the solid acid reagent region 5, and the specimen is developed by a capillary action to the sample pad 10, the sample pad 10 serving as the solid acid reagent region 5, or the sample pad 10 serving as both the neutralizing reagent region 6 and the solid acid reagent region 5, and the neutralizing reagent region 6, where a top laminate sheet or a PET sheet is present between the solid acid reagent region 5 and the neutralizing reagent region 6. Thus, a flow of the specimen from the solid acid reagent region 5 to the neutralized reagent region 6 is suppressed, and the specimen having once entered the neutralized reagent region 6 is prevented from flowing backward to the solid acid reagent region 5. Then, the specimen is developed horizontally and successively to the label region 2, the support 1 and the absorbent pad 7. In the solid acid reagent region 5, the nitrite mixed with the specimen reacts with the solid acid reagent on the solid acid reagent region 5 to generate free nitrous acid, so that an action of the nitrous acid allows a sugar chain antigen to be extracted from the specimen. The extracted sugar chain antigen is developed and moved together with an acidic developing solution to the neutralized reagent region 6, and the pH of the acidic developing solution containing the sugar chain antigen is neutralized and adjusted to the neutral range in the neutralizing reagent region 6. As a result, the sugar chain antigen is further developed and moved downstream under neutral conditions. In the label region 2, along with the development of the specimen sample, a label antigen is released into a solution and developed to the support 1. When a sugar chain antigen is present in the specimen sample, the sugar chain antigen is specifically captured by a capturing antibody at a detection region 3 of the support 1, and the sugar chain antigen causes a specific reaction with a labeled antibody to form a complex. This enables antibodies to construct a sandwich via the sugar chain antigen in the detection region 3, thereby measuring a labeled-antibody-sugar chain antigen complex in the detection region 3.

An immunochromatographic test kit of the present invention comprises a nitrite solution (R1) or an acid solution (R2) for suspending a specimen, and the kit is characterized by adding a substance to prevent a lack of sensitivity caused by a development defect or development delay, or a non-specific reaction, to the nitrite solution (R1) or the acid solution (R2) for suspending the specimen, even when a specimen with a high viscosity such as a pharynx swab specimen is used. Examples of such a substance include a surfactant, salts, N-acetyl-L-cysteine (NALC), a proteinaceous substance such as BSA (bovine serum albumin), phosphoric acid, sodium hydroxide, polyvinyl alcohol (PVA), and other chemical synthetic agents. The surfactant is not limited, and any of a non-ionic surfactant, a cationic surfactant and an anionic surfactant may be used. Specifically, examples thereof include a polyoxyethylene octyl phenyl ether such as Triton X-100 and Triton X-114; a quaternary ammonium compound such as benzalkonium chloride and benzethonium chloride; a polyoxyethylene sorbitan fatty acid ester such as Tween 20 and Tween 80; a polyoxyethylene alkyl ether such as Brij-35 and Brij-58; cholic acid having a steroid skeleton such as sodium cholate and deoxycholic acid; and an anionic surfactant such as sodium lauryl sulfate and sodium dodecylbenzenesulfonate. Among these surfactants, a polyoxyethylene octyl phenyl ether such as Triton X-100 and Triton X-114; and a quaternary ammonium compound such as benzalkonium chloride and benzethonium chloride are preferred. Examples of the salts include NaCl, NaBr, and others. Exemplary concentrations, at which the above exemplified compounds should be added, are shown below.
Triton X-100 (Tx-100): 0.5 to 5%, preferably 1 to 3%, and more preferably 2%
Benzalkonium chloride: 5 to 20%, preferably 5 to 15%, and more preferably 10%
Benzethonium chloride: 10 mM to 200 mM, preferably 50 to 150 mM, and more preferably 100 mM
Phosphate buffer solution: 100 to 500 mM, preferably 150 to 350 mM, and more preferably 250 mM
NaOH: 0.05 N to 3N, preferably 0.05 N to 2 N, and more preferably 0.1 N to 1 N
NALC: 10 mM to 200 mM, preferably 50 to 150 mM, and more preferably 100 mM
NaCl: 1 to 5 N, preferably 2 to 4 N, and more preferably 3N
BSA: 0.5 to 3%, preferably 0.5 to 2%, and more preferably 1%
NaBr: 10 mM to 200 mM, preferably 50 to 150 mM, and more preferably 100 mM

Among the above surfactants, in particular, PVA is highly effective, and PVA having a degree of saponification of 70 to 90 mol·% is preferred. In addition, the effect is observed at a PVA concentration of 0.01% to 5%, and the concentration of 0.01% to 0.05% is more preferred.

According to a method using the immunochromatographic test kit of the present invention, extraction of a sugar chain antigen in a specimen is carried out on an immunochromatographic test strip, and therefore, it is not necessary to extract the sugar chain antigen in the specimen before measurement using the immunochromatographic test strip, making it possible to measure the sugar chain antigen in the specimen by a single step.

In the method using the immunochromatographic test kit of the present invention, a biological sample used as a specimen is not particularly limited. Examples of such a biological sample include body fluid such as serum, plasma, blood, urine, feces, saliva, tissue fluid, spinal fluid or swab solutions, and diluted products thereof.

In the method using an immunochromatographic test kit of the present invention, a substance to be detected as an analyte is a sugar chain antigen, which can be measured by an immunoassay, namely, an assay utilizing an antigen-antibody reaction. Examples of the antigen include polysaccharides that are a sugar chain antigen present on the cell wall of bacteria extracted by a nitrous acid extraction treatment. Protozoa, fungi, bacteria, mycoplasma, rickettsia, chlamydia, virus, and others comprising the aforementioned substance can also be measured. According to the method using an immunochromatographic test strip of the present invention, whether or not a sugar chain antigen derived from protozoa, fungi, bacteria, mycoplasma, rickettsia, chlamydia, virus, etc. is contained in a biological sample of a subject can be confirmed. When such a sugar chain antigen is contained, it can be determined that the subject is affected by infection caused by protozoa, fungi, bacteria, mycoplasma, rickettsia, chlamydia, virus, etc. The presence or absence of infection with, for example, group A β-hemolytic streptococcus (*Streptococcus pyogenes*), *Escherichia coli*, Legionella, Campylobacter, etc. can be detected.

The kit of the present invention is not limited to the case wherein an immunochromatographic test strip is impregnated with a nitrite or an acid reagent, and a neutralizing reagent; but it is usable for a case wherein several reagents, in other words, two to N number of reagents that have different effects and should avoid a reaction during preservation are impregnated onto an immunochromatographic test strip.

### Examples

Hereinafter, the present invention will be more specifically described in the following examples. However, these examples are not intended to limit the scope of the present invention.

In the following examples, % represents w/v % unless otherwise specified.

### Example 1

### Example of Immunochromatographic Test Strip for Measuring Group A β-hemolytic Streptococcus

The following matters were used as a nitrate, a solid acid reagent and a neutralizing reagent.
Nitrite (liquid agent): 3.0 M sodium nitrite
Solid acid reagent (impregnated into the immunochromatographic test strip): 1.0 M citric acid
Neutralizing reagent (impregnated into the immunochromatographic test strip): 3.0 M Tris base (Trizma BASE)

### 1. Immobilization of anti-Streptococcus pyogenes (group A β-hemolytic streptococcus) antibody on nitrocellulose membrane (support)

A solution obtained by dilution of an anti-*Streptococcus pyogenes* antibody and an anti-rabbit IgG antibody were prepared. The anti-*Streptococcus pyogenes* antibody was linearly applied to a sample pad side of a nitrocellulose membrane backed with a PET film, and the anti-rabbit IgG antibody was linearly applied to an absorbent pad side thereof. Then, the nitrocellulose membrane was dried and an anti-*Streptococcus pyogenes* antibody-immobilized membrane was obtained. This membrane is referred to as an "antibody-immobilized membrane" in the present example.

### 2. Immobilization of anti-Streptococcus pyogenes antibody on colored polystyrene particles

Colored polystyrene particles were added to the diluted anti-*Streptococcus pyogenes* antibody solution; and after stirring, carbodiimide was added and the mixture was further stirred. A supernatant was removed by a centrifugal operation and then, the remaining was re-suspended in a buffer solution, so that an anti-*Streptococcus pyogenes* antibody-bound colored polystyrene particle suspension was obtained. These particles are referred to as "antibody-immobilized particles (colored polystyrene)" in the present example.

### 3. Application and drying of anti-Streptococcus pyogenes antibody-immobilized colored polystyrene particles

A predetermined amount of the antibody-immobilized colored polystyrene particle suspension produced in the above 2 was applied to a non-woven fabric, and it was dried. The obtained non-woven fabric is referred to as a "C. Pad" in the present example.

### 4. Application of neutralizing reagent (basic reagent)

The above neutralizing reagent (basic reagent) was applied to filter paper.

### 5. Production of solid acid reagent-impregnated non-woven fabric

The above solid acid reagent was applied to a non-woven fabric. Immediately after the application, it was dried, so that a solid acid reagent-impregnated non-woven fabric was obtained as a solid acid reagent-impregnated region.

### 6. Production of immunochromatographic test strip for detection of Streptococcus pyogenes

First, the antibody-immobilized membrane (support) was adhered to a portion at 20 mm from the downstream of a backing sheet of a strip.

An absorbent pad for absorbing a liquid was adhered downstream of the membrane.

Glass fiber (C. Pad) of 10 mm width, to which antibody-bound colored polystyrene particles were applied, was adhered upstream of the membrane, with an overlap of 2 mm with the membrane.

The pad impregnated with the neutralizing reagent (neutralizing reagent-impregnated region) was adhered with an overlap of 4 mm between the C. Pad and the neutralizing reagent-impregnated pad.

A top laminate sheet (60 mm), which was a PET sheet, was adhered to align with an upper part of the immunochromatographic test strip.

Finally, the pad impregnated with the solid acid reagent (solid acid reagent-impregnated region) was adhered above the pad impregnated with the neutralizing reagent to align with the upstream portion (lower end) of the immunochromatographic test strip such that the pad impregnated with the solid acid reagent and the pad impregnated with the neutralizing agent come into contact with each other with the top laminate sheet present therebetween.

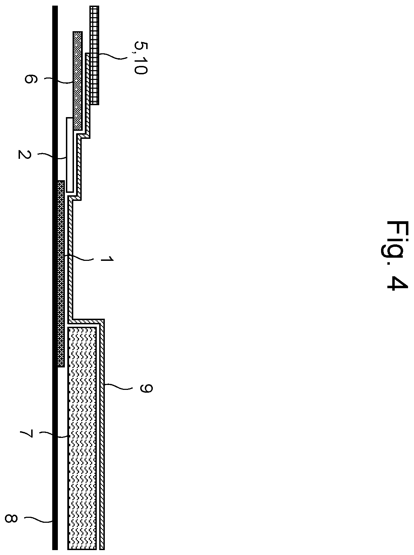

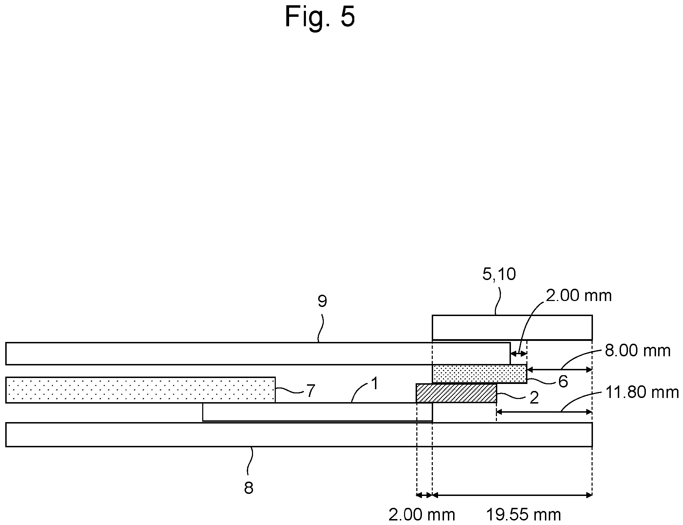

The structure of the immunochromatographic test strip thus produced is shown in Figure 4. In addition, Figure 5 is a schematic view also showing the respective distances (mm) between parts. In Figures 4 and 5, the pad impregnated with the solid acid reagent and the pad impregnated with the neutralizing reagent are depicted as if these pads are isolated with no contact even at a site where the top laminate is absent between the pads, but in actuality, both of the pads make contact with each other at the portion where the top laminate is not present.

### Example 2

The immunochromatographic test strip of Example 1 was placed in a test device case (a resin case for storing an immunochromatographic test strip). Using a solution containing only 3.0 M sodium nitrite solution as a control, prepared were solutions with respective conditions, under which Triton X-100, benzalkonium chloride, benzethonium chloride, phosphoric acid, sodium hydroxide, NALC, NaCl, PVA, BSA and NaBr were added to a 3.0 M sodium nitrite solution at concentrations shown in Table 1. A negative human nasal aspirate specimen that was known to cause a non-specific reaction was used, and the specimen was treated with a sodium nitrite solution and sodium nitrite solutions with respective substances described above (solution:specimen = 4:1) added, so that samples were prepared. 75 µL of each of the prepared samples was dropped and a determination was made after 5 minutes.

As a result, in comparison with the condition for control with no addition, a development start time was shortened for all of the conditions, and determination based on the lines was available at an earlier timing. In addition, the degree of a non-specific reaction (the intensity of color development of a non-specific line) was decreased compared to the control. In particular, under the conditions of addition of PVA, the development was improved and suppression of non-specific reactions was the most effective (Table 1).

**[Table 1]**

| | | Control | Condition 1 | Condition 2 | Condition 3 | Condition 4 | Condition 5 | Condition 6 | Condition 7 | Condition 8 | Condition 9 | Condition 10 | Condition 11 |
|---|---|---|---|---|---|---|---|---|---|---|---|---|---|
| | | No addition | 2% Tx-100 | 10% benzalkonium chloride | 100 mM benzethonium chloride hydrate | 250 mM phosphate buffer solution | 0.1N NaOH | 1.0N NaOH | 100mM NALC | 3.0M NaCl | 4% PVA | 1% BSA | 100mM NaBr |
| Development start time (sec) | | 60 | 45 | 120 | 55 | 100 | 60 | 60 | 40 | 30 | 60 | 60 | 48 |
| Color development of control line | 1min | N.D. | N.D. | N.D. | N.D. | N.D. | N.D. | N.D. | N.D. | N.D. | N.D. | N.D. | N.D. |
| | 2min | N.D. | + | + | + | + | + | + | ++ | ++ | ++ | N.D. | ++ |
| | 3min | N.D. | ++ | ++ | ++ | ++ | ++ | ++ | ++ | ++ | ++ | ++ | ++ |
| | 4min | N.D. | +++ | +++ | +++ | ++ | ++ | ++ | +++ | +++ | +++ | ++ | ++ |
| | 5min | + | +++ | +++ | +++ | ++ | ++ | ++ | +++ | +++ | +++ | ++ | ++ |
| Color development of test line | 1min | N.D. | N.D. | N.D. | N.D. | N.D. | N.D. | N.D. | N.D. | N.D. | N.D. | N.D. | N.D. |
| | 2min | ++ | + | N.D. | N.D. | N.D. | ± | ± | ± | + | - | N.D. | - |
| | 3min | ++ | + | ± | ± | ± | + | + | ± | + | - | ± | - |
| | 4min | ++ | + | + | ± | ± | + | + | - | + | - | ± | ± |
| | 5min | ++ | + | + | ± | ± | + | + | - | + | - | ± | ± |

| | | | | | | | | | | | | | |
|---|---|---|---|---|---|---|---|---|---|---|---|---|---|
| N.D.: Not Determined (impossible to determine due to no development of Latex) | | | | | | | | | | | | | |

### Example 3

The immunochromatographic test strip of Example 1 was placed in a test device case. As a sodium nitrite solution, prepared were (1) 2.0 M sodium nitrite solution (no PVA) and (2) 2.0 M sodium nitrite + 0.5% PVA. A human pharynx swab specimen was treated with each of sodium nitrite solutions to which viable cells of *Streptococcus pyogenes* were added, so that samples were prepared. 75 µL of the prepared sample was dropped and a determination was made after 5 minutes.

As a result, the condition with PVA shortened a development start time. In the case of a positive specimen, the positive result was available at an earlier timing, and a determination time period of 5 minutes resulted in a stronger line color development. In addition, when a negative specimen was tested, the frequency of occurrence of a non-specific reaction was decreased by about 4% (Table 2).

**[Table 2]**

| | | Specimen A | | Specimen B | | Specimen C | | Specimen D | | Specimen E | | Specimen F | | Specimen G | | Specimen H | |
|---|---|---|---|---|---|---|---|---|---|---|---|---|---|---|---|---|---|
| | | With PVA | No PVA | With PVA | No PVA | With PVA | No PVA | With PVA | No PVA | With PVA | No PVA | With PVA | No PVA | With PVA | No PVA | With PVA | No PVA |
| Development start time (sec) | | 160 | 180 | 120 | 135 | 60 | 90 | 90 | 120 | 60 | 75 | 60 | 100 | 120 | 240 | 120 | 120 |
| Color development of test line | 1min | N.D. | N.D. | N.D. | N.D. | N.D. | N.D. | N.D. | N.D. | N.D. | N.D. | N.D. | N.D. | N.D. | N.D. | N.D. | N.D. |
| | 2min | N.D. | N.D. | N.D. | N.D. | N.D. | N.D. | ++ | N.D. | ± | N.D. | + | N.D. | ± | N.D. | N.D. | N.D. |
| | 3min | N.D. | N.D. | ± | N.D. | + | ± | ++ | + | + | + | ++ | ++ | + | N.D. | N.D. | N.D. |
| | 4min | ++ | + | ++ | + | + | + | +++ | ++ | ++ | ++ | ++ | ++ | + | ± | ++ | + |
| | 5min | ++ | ++ | +++ | ++ | +++ | ++ | +++ | ++ | +++ | +++ | +++ | +++ | ++ | ++ | ++ | ++ |

| | | | | | | | | | | | | | | | | | |
|---|---|---|---|---|---|---|---|---|---|---|---|---|---|---|---|---|---|
| N.D.: Not Determined impossible to determine due to no color development of control line | | | | | | | | | | | | | | | | | |

### Reference Signs List

- 1: Support (including detection region)
- 2: Label region
- 3: Detection region
- 5: Solid acid reagent region
- 6: Neutralizing reagent region
- 7: Absorbent pad
- 8: Backing sheet
- 9: Top laminate sheet
- 10: Sample pad

### Industrial Applicability

Use of the immunochromatographic device of the present invention allows accurate detection of infection with group A β-hemolytic streptococcus.

All publications, patents, and patent applications cited in the present description are incorporated herein by reference in their entirety.

## Claims

1. An immunochromatographic test kit comprising:
(i) a nitrite solution or an acid solution; and
(ii) an immunochromatographic test strip for extracting and measuring a sugar chain antigen in a specimen, comprising: a sample pad to which the specimen mixed with the nitrite or acid solution is added; a label region comprising a labeled antibody obtained by labeling an antibody against the sugar chain antigen; and a detection region on which an antibody against the sugar chain antigen is immobilized, wherein an antibody-sugar chain antigen-labeled antibody complex is formed in the detection region to measure the sugar chain antigen, and wherein the immunochromatographic test strip has a region impregnated with a neutralizing reagent upstream of the label region, and further has, upstream of the region impregnated with the neutralizing reagent, a region impregnated with a solid acid reagent in case the specimen mixed with the nitrite is used, or a region impregnated with the nitrite in case the specimen mixed with the acid solution is used,
wherein the nitrite or acid solution comprises one or more selected from the group consisting of polyoxyethylene octyl phenyl ether, a quaternary ammonium compound, phosphoric acid, sodium hydroxide, N-acetyl-L-cysteine, NaCl, PVA, BSA and NaBr.

2. The immunochromatographic test kit according to claim 1, wherein the region impregnated with the solid acid reagent or the nitrite is present on the sample pad.

3. The immunochromatographic test kit according to claim 1 or 2, wherein the solid acid reagent is selected from the group consisting of malonic acid, malic acid, maleic acid, citric acid, and tartaric acid.

4. The immunochromatographic test kit according to any one of claims 1 to 3, wherein the neutralizing agent is tris(hydroxymethyl)aminomethane or sodium hydroxide.

5. The immunochromatographic test kit according to any one of claims 1 to 4, wherein the sugar chain antigen is a sugar chain antigen of protozoa, fungi, bacteria, mycoplasma, rickettsia, chlamydia, or virus.

6. The immunochromatographic test kit according to any one of claims 1 to 5, wherein the polyoxyethylene octyl phenyl ether is Triton X and the quaternary ammonium compound is benzalkonium chloride or benzethonium chloride.

7. A method for measuring a sugar chain antigen in a specimen by an immunochromatography method using the immunochromatographic test kit according to any one of claims 1 to 6, comprising:
mixing the specimen with a nitrous acid solution when the immunochromatographic test strip has a region impregnated with a solid acid reagent, or mixing the specimen with an acid solution when the immunochromatographic test strip has a region impregnated with a nitrite, and adding the mixture to a sample pad of the immunochromatographic test strip,
wherein, in the immunochromatography method, the sugar chain antigen is extracted from the specimen by the action of nitrous acid generated through a reaction of the nitrite with the solid acid reagent in the region impregnated with the solid acid reagent or the region impregnated with the nitrite, the acid solution containing the sugar chain antigen is neutralized in a region impregnated with a neutralizing reagent, and an antibody-sugar chain antigen-labeled antibody complex is formed in a detection region, and
wherein the immunochromatography method controls a speed or a direction of development of the specimen on the immunochromatographic test strip and controls treatments of the acid, the nitrite and the neutralizing reagent to detect sugar chain antigens in specimens.
